# FASCICULE DE BREVET EUROPEEN

(11) **EP 4 445 939 B1**
(45) Date de publication et mention de la délivrance du brevet: **17.12.2025**
(21) Numéro de dépôt: 24192676.5
(22) Date de dépôt: 01.03.2023
(51) Int. Cl.: A61M 16/00, A61M 16/10, A61M 16/12, A61M 16/20

(54) **APPAREIL DE DÉLIVRANCE DE NO À DEUX SORTIES DE GAZ**
NO-ABGABEVORRICHTUNG MIT ZWEI GASAUSLÄSSEN
NO DELIVERY APPARATUS WITH TWO GAS OUTLETS

(30) Priorité: 09.03.2022 FR 2202053
(43) Date de publication de la demande: 16.10.2024
(62) Demande divisionnaire de: 23159400.3
(73) Titulaire: INOSYSTEMS, 92160 Antony (FR)
(72) Inventeur: MARCHAL, Frédéric, 92160 Antony (FR)
(74) Mandataire: Air Liquide

(56) Documents cités:
- EP-A1- 2 581 103
- EP-A1- 3 233 171
- EP-A1- 3 906 955
- US-A- 5 558 083
- US-A1- 2015 320 951
- US-A1- 2017 095 634

## Description

La présente invention concerne un dispositif ou appareil de fourniture de monoxyde d'azote gazeux (NO) équipé d'un système de secours pour assurer une délivrance de NO, en mode normal, en mode secours en cas de panne ou en mode de ventilation manuelle, en particulier en cas de passage à une ventilation manuelle par ballon de ventilation ou d'insufflation manuelle (i.e. BAVU), et une installation d'administration de NO à un patient comprenant un tel dispositif ou appareil de fourniture de NO.

Le monoxyde d'azote inhalé ou NOi est un médicament gazeux couramment utilisé pour traiter des patients souffrant d'hypertension artérielle pulmonaire aiguë, en particulier les vasoconstrictions pulmonaires chez l'adulte ou l'enfant, y compris les nouveau-nés (PPHN), comme décrit par exemple par EP-A-560928 ou EP-A-1516639.

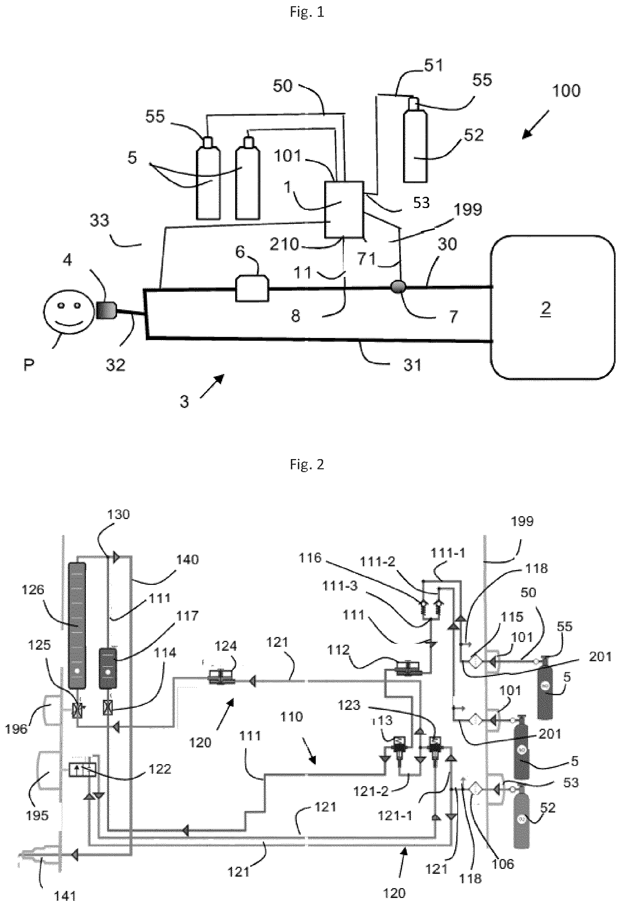

Une installation de mise en œuvre d'un traitement par NOi, couramment appelée installation d'administration de NO, comprend classiquement une ou des bouteilles de mélange NO/N₂ alimentant un dispositif de fourniture de NO qui délivre le mélange NO/N₂ à débit contrôlé, un appareil d'assistance respiratoire, aussi appelé ventilateur médical, pour fournir un gaz respiratoire contenant au moins 21 % vol. d'oxygène, tel un mélange O₂/N₂ ou de l'air, auquel est ajouté le NO (i.e. NO/N₂), des éléments de circuit, par exemple un ou plusieurs conduits flexibles, pour acheminer les flux gazeux entre ces différents équipements et jusqu'au patient, et une interface respiratoire, telle une sonde trachéale, pour fournir le mélange gazeux contenant le NO au patient. On peut prévoir aussi un humidificateur de gaz pour humidifier le mélange gazeux avant son administration au patient. Une telle installation est schématisée en Fig. 1.

Habituellement, le mélange NO/N₂ délivré par le dispositif de fourniture de NO est injecté dans le flux respiratoire contenant au moins 21% vol. d'oxygène (i.e. air ou mélange O₂/N₂) provenant du ventilateur médical avant d'être administré par inhalation au patient sous forme d'un mélange respiratoire final (i.e. mélange NO/N₂/O₂ ou NO/N₂/air) contenant généralement quelques dizaines ppmv de NO (ppm en volume) et au moins 21% vol. d'oxygène O₂, par exemple de l'ordre de 1 à 80 ppmv de NO, le reste étant essentiellement de l'azote (N₂).

Une telle installation d'administration de NO est utilisée en milieu hospitalier pour administrer le traitement par NOi et ainsi soigner les patients ayant besoin d'inhaler du NO pour traiter leur hypertension artérielle pulmonaire. Des exemples de telles installations d'administration de NO sont donnés par les documents WO-A-2012/094008, US-A-2015/320951, US-A-2015/273175, JP-A-H11192303, WO-A-02/40914 et US-A-2003/116159.

En cas de panne ou défaillance du dispositif de fourniture de NO ou du ventilateur médical et/ou en cas de nécessité de ventiler le patient avec un ballon de ventilation manuelle, sur demande du personnel soignant ou suite à une défaillance légère du dispositif, le dispositif de fourniture de NO doit pouvoir continuer à fournir le mélange NO/N₂ afin que le traitement du patient ne soit pas interrompu brutalement et ce, pour d'évidentes raisons de sécurité étant donné qu'une interruption brutale du traitement par NOi pourrait être fatale aux patients fragiles, en particulier les nouveau-nés souffrant de PPHN.

Pour assurer une délivrance de NO, même en cas de panne, de ventilation par ballon de ventilation manuelle ou autre, il est connu d'équiper le dispositif de fourniture de NO d'un système ou circuit secondaire, appelé circuit de secours, entièrement pneumatique conçu pour délivrer un débit d'O₂ réglable entre 0 et 20 L/min et un débit fixe de NO, par exemple de l'ordre de 230 mL/min de mélange NO/N₂, qui sont mélangés l'un à l'autre dans le dispositif de fourniture de NO avant d'être injectés dans un ballon de ventilation manuelle. L'oxygène est typiquement fourni par une source d'oxygène, telle une bouteille d'oxygène sous pression, venant se raccorder au dispositif de fourniture de NO. Ainsi, on peut citer EP-A-3233171 qui enseigne l'usage d'un tel circuit de secours.

Le passage en « mode secours » s'effectue après activation par l'utilisateur d'un moyen de sélection présent sur le dispositif de fourniture de NO, par exemple un bouton de sélection du « mode secours », dont l'activation va diriger les flux de gaz dans un circuit secondaire, i.e. de secours et/ou de ventilation manuelle du dispositif de fourniture de NO, tel celui illustré en Fig. 2.

Toutefois, une telle solution uniquement pneumatique n'est pas idéale car la concentration en NO du mélange gazeux obtenu (i.e. NO/N₂ + O₂) varie en fonction du débit d'oxygène réglé par l'utilisateur puisque le débit de NO est fixe. Or, avoir une concentration en NO qui varie en fonction du débit d'O₂ choisi, n'est pas souhaitable au plan thérapeutique car on doit pouvoir délivrer au patient une posologie donnée, c'est-à-dire une quantité de NO fixe correspondant à une concentration efficace pour traiter sa pathologie ou une dosage réduite en cas de nécessité de sevrage du patient, en particulier en mode de ventilation manuelle via un insufflateur manuel, c'est-à-dire un ballon de ventilation manuelle ou BAVU.

Au vu de cela, un problème est de proposer un dispositif de fourniture de NO amélioré, qui soit apte à fournir un mélange gazeux NO/N₂/O₂ à un patient que ce soit en mode de fonctionnement normal mais aussi en cas de panne ou de défaillance, ou encore lors d'un passage à une ventilation manuelle par BAVU (e.g. sur demande du personnel soignant ou suite à une défaillance légère), de préférence en observant la posologie souhaitée, c'est-à-dire un mélange gazeux NO/N₂/O₂ dont la composition n'est pas uniquement dépendante du débit d'oxygène.

Une solution de l'invention concerne un dispositif ou appareil de fourniture de NO (i.e. de monoxyde d'azote) comprenant :
- un circuit principal de gaz comprenant au moins une ligne principale de NO reliant fluidiquement au moins un port d'entrée de NO à un orifice de sortie principal pour acheminer un mélange NO/N₂ dudit au moins un port d'entrée de NO audit orifice de sortie principal, et
- un circuit de secours comprenant un circuit de NO de secours et un circuit d'O₂ de secours, dans lequel :
   - le circuit de NO de secours comprend une ligne de NO de secours alimentée en mélange NO/N₂ provenant dudit au moins un port d'entrée de NO, et
   - le circuit d'O₂ de secours comprend une ligne principale d'O₂ en communication fluidique avec un port d'entrée d'O₂, la ligne principale d'O₂ venant se raccorder fluidiquement à la ligne de NO de secours pour former une ligne commune en communication fluidique avec une sortie secondaire, aussi appelée sortie de secours, fournissant un mélange NO/N₂/O₂, en particulier de manière à pouvoir alimenter un ballon de ventilation manuelle (BAVU) ou analogue.

De plus, la ligne principale de NO comprend des moyens de contrôle de débit de NO/N₂ pilotés par des moyens de pilotage, et la ligne principale de NO est raccordée fluidiquement à la ligne de NO de secours, en aval des moyens de contrôle de débit de NO/N₂, par l'intermédiaire d'une première électrovanne à plusieurs voies.

Le dispositif de fourniture de NO comprend en outre un écran numérique d'affichage d'informations à commande tactile configuré pour afficher au moins une touche de sélection tactile. Ladite au moins une touche de sélection tactile affichée sur l'écran d'affichage est configurée pour permettre à l'utilisateur de fixer ou sélectionner une teneur en NO désirée comprise entre 1 et 80 ppmv. Ledit écran numérique est configuré pour afficher en outre la teneur en NO désirée.

Selon le mode de réalisation considéré, le dispositif ou appareil de fourniture de NO de l'invention peut comprendre l'une ou plusieurs des caractéristiques suivantes :
- les moyens de pilotage (i.e. dispositif de pilotage) sont alimentés en énergie électrique (i.e. courant électrique) par des moyens de fourniture d'énergie électrique,
- la ligne principale de NO est raccordée fluidiquement à la ligne de NO de secours par l'intermédiaire d'une première électrovanne à au moins 3 voies, de préférence à 3 voies ou 5 voies, avantageusement à 3 voies.
- la première électrovanne à plusieurs voies comprend au moins une voie d'entrée raccordée fluidiquement à la ligne principale de NO en aval des moyens de contrôle de débit, et une première voie de sortie reliée à la ligne principale de NO, en particulier à la portion aval de la ligne principale de NO comprenant l'orifice de sortie principal, et une seconde voie de sortie reliée à la ligne de NO de secours en amont de la ligne commune comprenant l'orifice secondaire.
- la première électrovanne est pilotée par les moyens de pilotage de manière à diriger le flux de mélange NO/N₂ provenant de moyens de contrôle de débit de NO/N₂ (202) (i.e. dispositif de contrôle de débit) :
   ▪ soit dans une portion aval de la ligne de NO de secours en communication fluidique avec la ligne commune en communication fluidique avec la sortie secondaire,
   ▪ soit dans une portion aval de la ligne principale de NO en communication fluidique avec l'orifice de sortie principal.
- la ligne de NO de secours comprend une vanne pneumatique permettant de contrôler la circulation du mélange NO/N₂ dans la ligne de NO de secours.
- la ligne de NO de secours comprend une seconde électrovanne commandée par les moyens de pilotage.
- la seconde électrovanne du circuit de NO de secours est normalement ouverte.
- la ligne principale d'O₂ comprend une première vanne de contrôle commandée par un moyen d'actionnement actionnable par un utilisateur pour contrôler la circulation du flux d'oxygène dans ladite ligne principale d'O₂.
- le moyen d'actionnement (i.e. dispositif d'actionnement) est configuré pour fournir un signal d'activation aux moyens de pilotage en réponse à un actionnement dudit moyen d'actionnement par l'utilisateur.
- la ligne principale d'O₂ comprend une seconde vanne de contrôle pneumatique agencée en aval de la première vanne de contrôle.
- les moyens de pilotage sont configurés pour contrôler les moyens de contrôle de débit de NO/N₂ de manière à régler le débit du mélange NO/N₂ dans le circuit principal de gaz.
- les moyens de pilotage sont configurés pour commander la première électrovanne, en réponse à la réception du signal d'activation délivré après actionnement du moyen d'actionnement par l'utilisateur, par exemple en cas de défaillance légère n'engendrant pas un arrêt total de fourniture d'énergie électrique et/ou d'arrêt de fonctionnement des moyens de pilotage, ou en particulier lorsque l'utilisateur souhaite opérer une ventilation manuelle par BAVU.
- les moyens de pilotage sont configurés pour commander la seconde électrovanne du circuit de NO de secours pour interrompre toute circulation de mélange NO/N₂ au travers de ladite seconde électrovanne.
- la première électrovanne à plusieurs voies est agencée sur la ligne principale de NO en aval des moyens de contrôle de débit de NO/N₂ et est en communication fluidique avec la ligne de NO de secours en aval de la vanne pneumatique et de la seconde électrovanne.
- la ligne principale d'O₂ comprend en outre des moyens de mesure de débit d'oxygène agencés en aval de la première vanne de contrôle et configurés pour fournir au moins une mesure de débit d'oxygène aux moyens de pilotage.
- la ligne principale d'O₂ comprend en outre des moyens de réglage de débit d'O₂ (i.e. dispositif de réglage de débit) agencés en aval des moyens de mesure de débit d'oxygène.
- il comprend des moyens de réglage de teneur en NO configurés pour permettre à un utilisateur de choisir une teneur en NO désirée et fournir ladite teneur en NO désirée aux moyens de pilotage.
- les moyens de pilotage sont configurés pour calculer un débit de mélange NO/N₂ à fournir correspondant à la teneur en NO désirée et piloter les moyens de contrôle de débit de NO/N₂ pour fournir ledit débit de mélange NO/N₂ calculé.
- les moyens de pilotage sont configurés pour calculer le débit de mélange NO/N₂ à fournir à partir de la teneur en NO désirée, du débit d'oxygène mesuré par les moyens de mesure de débit d'oxygène et d'une teneur en NO dans le mélange NO/N₂ acheminé par la ligne principale de NO.
- L'écran d'affichage numérique à commande tactile est un écran à dalle tactile.
- les moyens de réglage de teneur en NO comprennent au moins une touche de sélection tactile affichée sur l'écran d'affichage, de préférence des touches de sélection tactiles affichées sur l'écran d'affichage.
- la teneur en NO dans le mélange NO/N₂ acheminé par la ligne principale de NO est comprise entre 100 et 1000 ppmv.
- les moyens de pilotage sont en outre configurés pour, en l'absence d'actionnement du moyen d'actionnement par l'utilisateur, assurer un fonctionnement normal du dispositif en pilotant les moyens de contrôle de débit de NO/N₂ de manière à acheminer le mélange NO/N₂ par le circuit principal de gaz entre ledit au moins un port d'entrée de NO et l'orifice de sortie principal.
- la ligne principale de NO est reliée fluidiquement à deux ports d'entrée de NO agencés en parallèle.
- les moyens de pilotage sont configurés pour recevoir, lorsqu'ils sont alimentés en énergie électrique (i.e. courant électrique), le signal d'activation délivré après actionnement du moyen d'actionnement par l'utilisateur.
- la ligne de NO de secours et la ligne principale d'O₂ sont raccordées fluidiquement l'une à l'autre en un site de jonction situé en aval des moyens de réglage de débit d'O₂ de la ligne principale d'O₂ et en aval de la seconde électrovanne de la ligne de NO de secours.
- les moyens de contrôle de débit d'O₂ comprennent un capteur de débit ou un capteur de pression différentiel.
- la ligne principale d'O₂ est configurée pour coopérer pneumatiquement avec la vanne pneumatique par l'intermédiaire d'un tronçon de conduit reliant fluidiquement la ligne principale d'O₂ en aval de la seconde vanne de contrôle, à ladite vanne pneumatique.
- la première vanne de contrôle est de type tout ou rien.
- la première vanne de contrôle est configurée pour contrôler la circulation du flux d'oxygène dans la ligne principale d'O₂.
- la première vanne de contrôle est commandée par un moyen d'actionnement actionnable par l'utilisateur, tel un personnel soignant, lorsqu'il souhaite mettre en marche ou stopper le circuit de secours.
- le moyen d'actionnement comprend un sélecteur rotatif ou analogue actionnable par l'utilisateur.
- les moyens de réglage de teneur en NO comprennent au moins une touche de sélection à actionnement tactile affichée sur l'écran d'affichage, i.e. une ou des touches de sélection tactiles, et configurée pour permettre à un utilisateur de fixer ou sélectionner une teneur en NO désirée, de préférence des touches de sélection tactiles affichées sur l'écran d'affichage. Autrement dit, la sélection ou la fixation de la concentration en NO désirée se fait donc par appui digital de la part de l'utilisateur sur la ou les touches de sélection à actionnement tactile affichées sur l'écran d'affichage.
- les moyens de pilotage sont configurés pour commander le ou les affichages sur l'écran d'affichage.
- les moyens de pilotage comprennent au moins un (micro)processeur, par exemple un microcontrôleur.
- les moyens de pilotage comprennent au moins une carte électronique comprenant ledit au moins un microprocesseur.
- les moyens de pilotage comprennent au moins un (micro)processeur mettant en œuvre au moins un algorithme, par exemple de traitement de données, de calcul ou autre.
- les moyens de pilotage sont en outre configurés, en réponse à la réception du signal d'activation, pour agir sur la seconde électrovanne pour interrompre toute circulation de mélange NO/N₂ dans la ligne de NO de secours.
- la première électrovanne est agencée sur la ligne principale de NO en aval des moyens de contrôle de débit de NO/N₂ et sur la ligne de NO de secours du circuit de NO de secours en aval de la vanne pneumatique.
- la première électrovanne à plusieurs voies coopère avec la ligne de NO de secours via un conduit de liaison reliant la ligne de NO de secours à l'une des voies de la première électrovanne.
- la première électrovanne est agencée sur la portion de conduit aval de la ligne principale de NO, entre les moyens de contrôle de débit de NO/N₂ et l'orifice de sortie principal.
- la première électrovanne est commandée par les moyens de pilotage pour autoriser ou stopper tout passage de mélange NO/N₂ provenant des moyens de contrôle de débit de NO/N₂.
- le site de jonction est situé en aval de la première électrovanne.
- la ligne de NO de secours comprend un dispositif à orifice calibré agencé en aval de la vanne pneumatique.
- un premier régulateur de pression est agencé sur la ligne de NO de secours en amont de la vanne pneumatique.
- un second régulateur de pression est agencé sur la ligne principale d'O₂ entre la seconde vanne de contrôle pneumatique et les moyens de mesure de débit d'oxygène.
- un dispositif indicateur de débit de NO est agencé sur la ligne de NO de secours en amont du site de jonction de la ligne principale d'O₂ et de la ligne de NO de secours.
- un dispositif indicateur de débit d'O₂ est agencé sur la ligne principale d'O₂, en aval du dispositif ou moyens de réglage de débit d'O₂.
- le dispositif indicateur de débit d'O₂ comprend un rotamètre à bille.
- les moyens de réglage de débit d'O₂ comprennent un disque rotatif à orifices calibrés ou analogue.
- les moyens de réglage de débit d'O₂ sont configurés pour ajuster le débit du flux d'O₂ entre 5 et 20 L/min.
- il comprend un moyen de sélection de débit coopérant avec les moyens de réglage de débit d'O₂ pour sélectionner un débit d'O₂ désiré.
- le moyen de sélection de débit est actionnable par l'utilisateur.
- le moyen de sélection de débit comprend un bouton rotatif ou analogue.
- selon un autre mode de réalisation, le moyen de sélection de débit comprend une touche ou analogue affichée sur un écran d'affichage pour opérer une sélection du débit d'O₂.
- il comprend des moyens de mémorisation de données, par exemple une mémoire informatique ou analogue.
- il comprend un boitier rigide, dans lequel sont agencés tout ou partie des éléments du dispositif de fourniture de NO.
- la ligne principale d'O₂ est configurée pour coopérer pneumatiquement avec la vanne pneumatique pour autoriser le passage du mélange NO/N₂ dans la ligne de NO de secours, après actionnement du moyen d'actionnement par l'utilisateur, c'est-à-dire en réponse à un actionnement par l'utilisateur du moyen d'actionnement.
- les moyens de pilotage sont en outre configurés pour, en l'absence d'actionnement du moyen d'actionnement par l'utilisateur, assurer un fonctionnement normal du dispositif en pilotant les moyens de contrôle de débit de NO/N₂ de manière à acheminer le mélange NO/N₂ par le circuit principal de gaz entre ledit au moins un port d'entrée de NO et l'orifice de sortie principal.
- les touches de sélection tactile permettent à l'utilisateur d'opérer un choix parmi plusieurs teneurs en NO proposées, i.e. de fixer ou sélectionner une teneur en NO désirée, par exemple parmi plusieurs teneurs en NO prédéfinies.
- alternativement, les touches de sélection tactile comprennent des touches « + » et « - » permettant d'incrémenter ou de décrémenter une valeur de NO donnée par palier, par exemple de 1 ppmv en 1 ppmv, ou autre incrément (e.g. de 2 en 2, 3 en 3, ... ou 5 en 5 ppmv).
- ladite teneur en NO désirée qui est choisie ou sélectionnée par l'utilisateur par action sur ladite au moins une touche de sélection tactile est fournie aux moyens de pilotage, de préférence par action digitale.
- l'écran d'affichage est à affichage en couleurs ou en noir et blanc.
- les moyens de fourniture d'énergie électrique, i.e. d'alimentation en courant électrique, comprennent des moyens de raccordement électrique au secteur (110/220V), par exemple un câble électrique muni d'une prise électrique ou analogue, et/ou une batterie électrique, de préférence rechargeable.
- l'écran d'affichage est porté par le boitier.
- la sortie secondaire, i.e. une sortie de secours, tel un port ou orifice de sortie, est configurée pour être raccordée fluidiquement à un insufflateur manuel, tel un ballon de ventilation manuelle ou BAVU, notamment via un conduit flexible de raccordement fluidique, i.e. tuyau souple ou analogue.
- la ligne principale de NO comporte au moins une portion de conduit amont, de préférence deux portions de conduit amont agencées en parallèle.
- le circuit de NO de secours est alimenté en NO/N₂ par ladite au moins une portion de conduit amont de la ligne principale de NO, de préférence les deux portions de conduit amont agencées en parallèle.
- le circuit de NO de secours est raccordé fluidiquement à ladite au moins une portion de conduit amont, de préférence aux deux portions de conduit amont agencées en parallèle, via au moins un tronçon de canalisation d'entrée de NO, de préférence deux tronçons de canalisation d'entrée de NO en parallèle venant se raccorder fluidiquement aux deux portions de conduit amont.
- le circuit de NO de secours vient se raccorder fluidiquement à la ligne principale de NO, en amont des moyens de contrôle de débit de NO/N₂, en particulier auxdites portions de conduit amont de la ligne principale de NO, de manière à être alimenté en NO/N₂.
- le ou les deux tronçons de canalisation d'entrée de NO sont alimentés en NO/N₂ par le ou les deux ports d'entrée de NO.

Selon un autre aspect, l'invention concerne aussi une installation d'administration de gaz thérapeutique contenant du NO, à un patient (P) comprenant un dispositif de fourniture de NO selon l'invention, en particulier tel que décrit ci-dessus, alimenté en mélange NO/N₂ par au moins un récipient de gaz sous pression et en oxygène par un récipient d'oxygène sous pression, ledit dispositif de fourniture de NO fournissant :
- soit un mélange NO/N₂ via l'orifice de sortie principal (210), à un circuit de gaz respiratoire (3) raccordé à un ventilateur médical (2),
- soit un mélange NO/N₂/O₂ via l'orifice secondaire (141), à un insufflateur manuel ou BAVU.

Selon le mode de réalisation considéré, l'installation d'administration de gaz thérapeutique de l'invention peut comprendre l'une ou plusieurs des caractéristiques suivantes :
- ledit au moins un récipient de gaz sous pression contient le mélange NO/N₂.
- ledit au moins un récipient de gaz sous pression contient un mélange NO/N₂ contenant de 100 à 1000 ppmv de NO, le reste étant de l'azote.
- le récipient d'oxygène sous pression contient de l'oxygène médical.
- le ou les récipients sont des bouteilles de gaz.
- le ou les récipients de gaz contiennent un mélange NO/N₂ ou de l'oxygène médical à une pression d'au moins 150 bar, voire au moins 180 bar.
- le circuit de gaz respiratoire comprend une branche inspiratoire et une branche expiratoire.
- la branche inspiratoire et la branche expiratoire sont reliées fluidiquement l'une à l'autre via une pièce de jonction, telle une pièce en Y.
- la pièce de jonction est raccordée fluidiquement à une interface respiratoire, telle une sonde trachéale ou un masque respiratoire.
- la branche inspiratoire et la branche expiratoire comprennent des tuyaux flexibles.
- un humidificateur de gaz est agencé sur le circuit de gaz respiratoire, en particulier sur la branche inspiratoire.
- la branche inspiratoire comprend un capteur de débit relié électriquement au dispositif de fourniture de NO, en particulier aux moyens de pilotage.
- le capteur de débit est agencé sur la branche inspiratoire en amont du site d'injection de NO.
- le capteur de débit est du type massique ou à différentiel de pression.
- une ligne de prélèvement de gaz relient fluidiquement le dispositif de fourniture de NO au circuit de gaz respiratoire, de préférence à proximité de la pièce de jonction, i.e. la pièce en Y.
- le ventilateur médical et le dispositif de fourniture de NO sont alimentés électriquement par des moyens de fourniture d'énergie électrique, c'est-à-dire au moins une source de courant électrique, en particulier des moyens de raccordement électrique au secteur (110/220V), par exemple un câble électrique muni d'une prise électrique ou analogue, et/ou une batterie électrique, de préférence rechargeable.
- la sortie secondaire, i.e. sortie de secours, du dispositif de fourniture de NO est raccordée fluidiquement à un ballon d'insufflation manuel, de préférence via un conduit de raccordement, tel un tuyau flexible.

Selon un autre aspect (non-revendiqué), la présente divulgation concerne aussi une méthode de traitement d'une personne, appelée un patient, souffrant d'une pathologie ou d'une condition médicale engendrant une hypertension artérielle pulmonaire aiguë, en particulier des vasoconstrictions pulmonaires chez l'adulte, l'adolescent ou l'enfant, y compris les nouveau-nés et les bébés, par exemple pour traiter une hypertension pulmonaire persistante du nouveau-né (PPHN) chez un nouveau-né, bébé, enfant ou analogue, ou une hypertension pulmonaire chez une personne subissant une chirurgie cardiaque, dans laquelle on met en oeuvre une administration par inhalation audit patient en ayant besoin, d'un mélange gazeux contenant de l'oxygène (de préférence >21%vol), de l'azote et du NO (de préférence < 100 ppmv), ledit mélange gazeux étant fourni par une installation d'administration de gaz thérapeutique selon l'invention comprenant un dispositif ou appareil de fourniture de NO selon l'invention, en particulier comme décrit ci-avant et/ou ci-après, lequel dispositif de fourniture de NO est alimenté en mélange NO/N₂ par au moins un récipient de gaz sous pression, de préférence plusieurs récipients de gaz sous pression 5, telles des bouteilles de NO/N₂, et en oxygène par au moins un récipient d'oxygène sous pression, telle une bouteille d'O₂, et lequel dispositif de fourniture de NO pouvant fournir : soit un mélange NO/N₂, via l'orifice de sortie principal, au circuit de gaz respiratoire raccordé à un ventilateur médical de l'installation d'administration de gaz thérapeutique selon l'invention, soit un mélange NO/N₂/O₂ via l'orifice secondaire, à un insufflateur manuel, en particulier un ballon d'insufflation manuel ou BAVU.

L'invention va maintenant être mieux comprise grâce à la description détaillée suivante, faite à titre illustratif mais non limitatif, en référence aux figures annexées parmi lesquelles :
Fig. 1 représente un mode de réalisation d'une installation d'administration de gaz thérapeutique à un patient P incorporant un dispositif de fourniture de NO,
Fig. 2 schématise un mode de réalisation du circuit de secours d'un dispositif de fourniture de NO selon l'art antérieur,
Fig. 3 schématise un mode de réalisation de l'architecture interne d'un dispositif de fourniture de NO selon la présente invention, et
Fig. 4 est un schéma détaillé des moyens de contrôle de débit de NO/N₂ du dispositif de Fig. 3.

Fig. 1 schématise un mode de réalisation d'une installation 100 d'administration de gaz thérapeutique, i.e. un mélange gazeux à base de NO, à un patient P incorporant un dispositif de fourniture de NO 1, tel que celui selon la présente invention.

Plus précisément, elle comprend ici deux récipients de gaz sous pression 5, agencés en parallèle, contenant chacun un mélange gazeux de NO et d'azote (N₂), i.e. un mélange NO/N₂, contenant typiquement de 250 à 1000 ppmv de NO et de l'azote (N₂) pour le reste conditionné à une pression pouvant atteindre 180 bar ou plus, par exemple un mélange NO/N₂, contenant 450 ppmv ou 800 ppmv de NO. De tels récipients de gaz 5 sont couramment appelés bouteilles de NO 5.

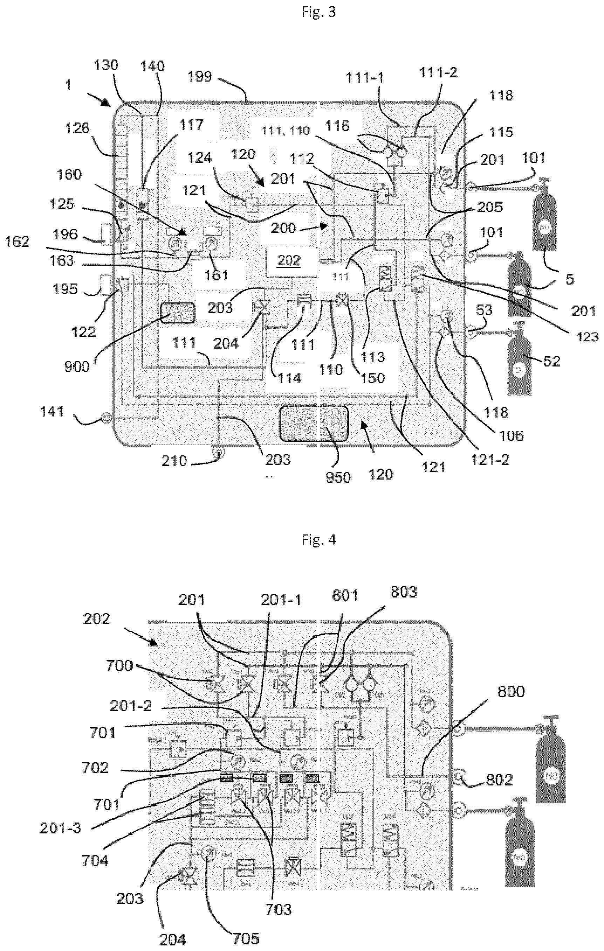

Les bouteilles de NO 5 fournissent le mélange NO/N₂ à un dispositif de fourniture de NO 1, tel celui selon l'invention dont l'architecture interne est illustrée sur Fig. 3 et Fig. 4. Elles sont reliées fluidiquement au dispositif de fourniture de gaz 1 par des lignes 50 de fourniture de NO, i.e. des canalisations de gaz, tels des tuyaux flexibles ou analogue. Chaque ligne 50 de fourniture de NO est reliée à un port d'entrée de NO 101 du dispositif de fourniture de NO 1 pour alimenter un circuit principal de gaz 200 interne au boîtier 199 du dispositif de fourniture de NO 1 (cf. Fig. 3).

Le dispositif de fourniture de NO 1 comprend aussi un port d'entrée d'oxygène 53 relié fluidiquement, via une ligne d'amenée d'oxygène 51, tel un tuyau flexible ou analogue, à une source d'oxygène par exemple un récipient d'oxygène sous pression 52, typiquement une bouteille d'O₂ ou, alternativement, le réseau hospitalier, i.e. une canalisation d'amenée d'oxygène agencée dans le bâtiment hospitalier où est soigné le patient P.

Les bouteilles de NO 5 et la bouteille d'O₂ 52 sont équipées d'un robinet de distribution de gaz 55, de préférence intégrant des moyens de détente de gaz, c'est-à-dire un RDI ou robinet à détendeur intégré, de manière à pouvoir contrôler le débit et/ou la pression du gaz qu'elles délivrent. Le robinet de distribution de gaz 55 est préférentiellement protégé contre les chocs par un capotage de protection.

Par ailleurs, l'installation 100 comprend aussi un ventilateur médical 2, c'est-à-dire un appareil d'assistance respiratoire, fournissant un flux de gaz respiratoire contenant au moins 21% vol. d'oxygène, tel de l'air ou un mélange oxygène/azote (N₂/O₂), au patient P.

Le ventilateur médical 2 est fluidiquement relié au patient P via un circuit de gaz 3 respiratoire qui est ici à deux branches respiratoires 30, 31 étant donné qu'il comprend une branche inspiratoire 30, c'est-à-dire une ligne d'alimentation en gaz, servant à amener le gaz respiratoire au patient P et une branche expiratoire 31 sevrant à récupérer le gaz enrichi en CO₂ expiré par le patient P.

Les deux branches respiratoires 30, 31 sont typiquement des tuyaux flexibles en polymère ou analogue. Les deux branches respiratoires 30, 31 sont, d'une part, raccordées au ventilateur médical 2 et, d'autre part, reliées l'une à l'autre au niveau d'une pièce de jonction 32, typiquement une pièce en Y, laquelle est en communication fluidique avec une interface respiratoire 4 fournissant le gaz au patient P, telle une sonde trachéale ou autre.

Bien entendu, le ventilateur médical 2 et le dispositif de fourniture de NO 1 sont normalement alimentés électriquement par une (des) source de courant électrique, en particulier leurs composants nécessitant de l'énergie électrique pour fonctionner, en particulier les moyens de pilotage 900 du dispositif de fourniture de NO 1 et le système de commande du ventilateur médical 2, i.e. carte électronique à microprocesseur(s), ou tout autre composant, notamment la turbine interne motorisée qui fournit le flux d'air ou analogue, i.e. le gaz respiratoire. La source de courant électrique peut être le secteur (110/220V) et/ou une batterie électrique, de préférence rechargeable.

Comme on le voit, le dispositif de fourniture de NO 1 permet d'injecter le mélange NO/N₂ dans la branche inspiratoire 30, via un conduit d'injection de NO 11 débouchant dans la branche inspiratoire 30 en un site d'injection 8, de manière à y opérer un mélange du flux de NO/N₂ et du flux de gaz respiratoire contenant au moins 21% d'O₂, i.e. air ou de mélange oxygène/azote, délivré par le ventilateur médical 2.

Le dispositif de fourniture de NO 1 comprend un orifice de sortie principal 210 situé au débouché de son circuit principal de gaz 200 par lequel le flux de NO/N₂ sort du boitier 199 du dispositif de fourniture de NO 1 et pénètre dans le conduit d'injection de NO 11. Le conduit d'injection de NO 11 est raccordé fluidiquement à l'orifice de sortie principal 210, par exemple via un connecteur ou analogue.

Le mélange gazeux thérapeutique obtenu contient donc de l'oxygène (>21% vol.), de l'azote et une concentration de NO variable et ajustable, typiquement comprise entre 1 et 80 ppmv, du fait de la dilution de produisant lors du mélange des flux gazeux. Bien entendu, des impuretés inévitables peuvent se trouver dans le gaz mais elles ne sont pas souhaitées, en particulier quand le flux de gaz provenant du ventilateur 2 est de l'air atmosphérique plutôt qu'un mélange O₂/N₂.

Avantageusement, on prévoit en outre un humidificateur de gaz 6 agencé ici sur la branche inspiratoire 30 en aval du site d'injection 8 servant à humidifier le flux de gaz thérapeutique, e.g. mélange NO/N₂/O₂, par addition de vapeur d'eau, avant qu'il ne soit inhalé par le patient P, ce qui permet d'éviter ou limiter l'assèchement des voies respiratoires du patient P pendant son traitement par inhalation du gaz. Selon un autre mode de réalisation, l'humidificateur de gaz 6 pourrait aussi être agencé en amont du site d'injection 8. Selon le cas, la branche expiratoire 31 servant à recueillir les gaz expirés riches en CO₂ peut comprendre un ou d'autres composants optionnels, comme par exemple un dispositif d'élimination du CO₂, i.e. un piège à CO₂, tel un bac à chaud ou autre, permettant d'éliminer le CO₂ présent dans les gaz expirés par le patient, un filtre ou autre.

Comme visible sur Fig. 1, il est aussi prévu sur la branche inspiratoire 30, en amont du site d'injection 8, un capteur de débit 7, par exemple du type massique ou à différentiel de pression, relié au dispositif de fourniture de NO 1, notamment aux moyens de pilotage 900 dudit dispositif de fourniture de NO 1, via une ligne de mesure de débit 71 de gaz respiratoire servant à mesurer le débit de gaz provenant du ventilateur 2 au sein de la branche inspiratoire 30. Déterminer ce débit du ventilateur (Qv) permet notamment de réguler le passage du NO au travers du dispositif de fourniture de NO 1, en particulier de pouvoir choisir le débit de mélange NO/N₂ à injecter en fonction de la teneur en NO désirée, de la composition du mélange NO/N₂ provenant des bouteilles et du débit de gaz (i.e. air ou air/O₂) provenant du ventilateur 2.

Par ailleurs, on peut prévoir aussi une ligne de prélèvement 33 de gaz reliant fluidiquement le dispositif de fourniture de NO 1 au circuit de gaz respiratoire 3, de préférence à proximité de la pièce en Y 32, par exemple à environ 10 à 20 cm en amont de la pièce en Y 32, servant à prélever des échantillons de gaz et à vérifier leur conformité avec le mélange gazeux souhaité devant être administré au patient P.

Plus précisément, le dispositif de fourniture 1 de NO comprend un circuit principal de gaz 200 interne pour acheminer le mélange NO/N₂ entrant par le ou les ports d'entrée de gaz 101 jusqu'au conduit d'injection de NO 11. Ce circuit principal de gaz 200 comprend des moyens de contrôle de débit 202 de NO/N₂ (cf. Fig. 3 et Fig. 4), telles des valves, des orifices calibrés..., pilotés par les moyens de pilotage 900 du dispositif de fourniture 1 de NO, typiquement un (ou des) microprocesseur agencé sur une carte électronique, dont le fonctionnement est expliqué ci-après. Tous ces composants sont agencés dans un boitier 199, c'est-à-dire une carcasse externe rigide.

De plus, le dispositif de fourniture 1 de NO comprend un circuit de secours 110, appelé circuit de « backup », conçu pour délivrer un débit d'O₂ réglable et un débit fixe de NO afin de pouvoir assurer une fourniture de NO, même en cas de panne ou autre, comme détaillé ci-après.

Ainsi, Fig. 2 schématise un mode de réalisation du circuit de secours 110 ou circuit secondaire d'un dispositif de fourniture 1 de NO classique. Il convient avant tout de préciser que, sur Fig. 2, le circuit de gaz principal 200 du dispositif de fourniture de NO 1, qui achemine le mélange NO/N₂ durant le fonctionnement normal du dispositif 1, c'est-à-dire hors panne ou analogue, n'est pas représenté afin de ne pas alourdir inutilement le schéma et compliquer sa compréhension. Néanmoins, ce circuit de gaz principal 200, qui est agencé dans le boitier 199, comprend, comme représenté celui illustré sur Fig. 3 ou Fig. 4, une ligne principale de NO 201, 203 comportant au moins une portion de conduit amont 201, à savoir ici deux portions de conduit amont 201 agencées en parallèle, puisque 2 bouteilles de NO 5 sont raccordées au dispositif de fourniture de NO 1, comme illustré sur Fig. 2 à Fig. 4, et par ailleurs au moins une portion aval 203.

Comme on le voit, le circuit de secours 110, 120, dont le fonctionnement peut être entièrement pneumatique, qui est aussi agencé dans le boitier 199, comprend un circuit de NO de secours 110 et un circuit d'O₂ 120 de secours qui comprennent chacun des canalisations, passages ou conduits de gaz permettant de véhiculer les différents gaz ou mélanges gazeux dans le boitier 199.

Le circuit de NO 110 de secours est alimenté en NO/N₂ par au moins un tronçon de canalisation d'entrée de NO 111-1, 111-2, à savoir ici deux tronçons de canalisation d'entrée de NO 111-1, 111-2 agencés en parallèle, eux-mêmes alimentés par les deux ports d'entrée de NO 101 auxquels sont connectées les bouteilles de NO 5, via la ligne d'alimentation 50, comme expliqué ci-avant.

Le ou les tronçons de canalisation d'entrée 111-1, 111-2, viennent se raccorder fluidiquement aux portions de conduit amont 201 (partiellement représentées sur Fig. 2) en aval d'un filtre 115 situé immédiatement en aval de chaque port d'entrée de NO 101 dans la ou les portions de conduit amont 201, comme détaillé en Fig. 3.

Un clapet anti-retour 116 est agencé dans chaque tronçon de canalisation d'entrée 111-1, 111-2. Ces deux tronçons de canalisation d'entrée 111-1, 111-2 se rejoignent en aval des clapets anti-retour 116 pour former une ligne de NO de secours 111 commune, à partir d'un site de jonction 111-3.

Chaque portion de conduit amont 201 comprend en outre un moyen de mesure de pression 118, tel qu'un capteur de pression à jauge de contrainte (*silicon pressure sensor* en anglais) ou analogue, servant à vérifier que l'existence d'une pression dans la portion de conduit amont 201 reflétant la présence d'une bouteille connectée et donc aussi de vérifier la quantité de gaz qu'elle contient afin de décider s'il faut la changer (si bouteille quasiment vide) et basculer sur l'autre bouteille de gaz. Ledit moyen de mesure de pression 118 est situé en aval du filtre 115.

Bien entendu, selon un autre mode de réalisation, le dispositif 1 peut ne comprendre qu'un seul port d'entrée de NO 101 et donc une seule portion de conduit amont 201 et un seul tronçon de canalisation d'entrée 111-1. Dans ce cas, seule une ligne d'amenée d'oxygène 51, tel un tuyau flexible ou analogue, peut y être raccordée fluidiquement, laquelle est alimentée en mélange NO/N₂ par une ou plusieurs bouteilles de NO 5.

La ligne de NO de secours 111 permet de véhiculer le mélange NO/N₂ sous pression provenant, via les tronçons de canalisation d'entrée 111-1, 111-2, des portions de conduit amont 201 alimentées par les bouteilles de NO 5 délivrant le mélange NO/N₂ à une pression de l'ordre de 3 à 6 bar relatif en sortie du RDI 55.

On voit par ailleurs que la ligne de NO de secours 111 comprend en outre un premier régulateur de pression 112, en aval du site de jonction 111-3, pour réduire ou contrôler la pression du mélange NO/N₂, par exemple pour délivrer une pression réduite égale à environ 3,2 bar relatif, et une vanne pneumatique 113 permettant de contrôler la circulation du mélange NO/N₂. Laquelle vanne pneumatique 113 est située en aval du premier régulateur de pression 112, en considérant le sens de circulation du gaz, sachant que le mélange NO/N₂ gazeux circule dans le sens allant de chaque port d'entrée de gaz 101 vers le régulateur de pression 112.

L'ouverture de la vanne pneumatique 113, donc le passage du flux de NO/N₂, est commandée par la pression du flux d'oxygène amené par la ligne principale d'O₂ 121 via le second tronçon de conduit 121-2, comme expliqué ci-après.

En aval de la vanne pneumatique 113, la ligne de NO de secours 111 comprend un dispositif à orifice calibré 114 ou analogue permettant de réguler le débit gazeux, i.e. du mélange NO/N₂, et un dispositif indicateur de débit de NO 117 permettant de contrôler que le débit de mélange NO/N₂ est bien égal à la valeur fixe escomptée, par exemple de l'ordre de 230 mL/min.

En outre, le circuit d'oxygène 120 de secours comprend quant à lui une ligne principale d'O₂ 121 servant à véhiculer l'oxygène dans le boitier 199, qui est alimentée par le port d'entrée d'O₂ 102 auquel est reliée la bouteille d'O₂ 52, via la ligne d'alimentation 51 qui amène l'oxygène gazeux à une pression typiquement comprise entre 3 et 6 bar.

La ligne principale d'O₂ 121 vient se raccorder en un site de jonction 130, à la ligne de NO de secours 111, en aval du dispositif indicateur de débit de NO 117 en y formant une ligne commune 140, de manière à opérer un mélange du flux d'oxygène à débit ajustable, comme expliqué ci-après, et du flux de NO/N₂ qui a un débit fixe de l'ordre de 230 mL/min par exemple.

Comme on le voit sur Fig. 2, la ligne principale d'O₂ 121 comprend elle aussi un filtre 106, agencé immédiatement en aval du port d'entrée d'O₂ 102, ainsi qu'une première vanne de contrôle 122 et une seconde vanne de contrôle 123 pneumatique. Elle comprend aussi un moyen de mesure de pression 118, tel un capteur de pression à jauge de contrainte, comme expliqué ci-avant.

La première vanne de contrôle 122, typiquement de type tout ou rien, permet de contrôler la circulation du flux d'oxygène dans la ligne principale d'O₂ 121. Elle est commandée par un moyen d'actionnement 195, tel un sélecteur rotatif, un bouton poussoir, une touche de sélection ou analogue, actionnable par l'utilisateur, tel un personnel soignant, lorsqu'il souhaite mettre en marche ou stopper le circuit de secours 110, 120.

Un premier tronçon de conduit 121-1 vient se raccorder fluidiquement à la ligne principale d'O₂ 121 entre le port d'entrée d'O₂ 102, en particulier en aval du filtre 106, et la première vanne de contrôle 122. Le premier tronçon de conduit 121-1 permet de piloter pneumatiquement la seconde vanne de contrôle 123 pneumatique.

Tant que la première vanne de contrôle 122 est fermée, la pression gazeuse s'exerçant dans la portion de la ligne principale d'O₂ 121 située en amont de la première vanne de contrôle 122 et donc aussi dans le premier tronçon de conduit 121-1 va agir sur la seconde vanne de contrôle 123 pneumatique en la maintenant fermée, ce qui va empêcher tout passage d'oxygène au travers de cette seconde vanne de contrôle 123.

Lorsque l'utilisateur actionne le moyen d'actionnement 195, la première vanne de contrôle 122 s'ouvre et permet alors le passage du gaz en direction de la seconde vanne de contrôle 123. Du fait de la pression de l'oxygène arrivant à la seconde vanne de contrôle 123, il va s'opérer une force suffisante pour permettre d'ouvrir la vanne de contrôle 123 du fait de la pression apportée par le premier tronçon de conduit 121-1, ce qui va alors ouvrir la seconde vanne de contrôle 123 et laisser alors passer l'oxygène dans la partie aval du circuit d'oxygène 120 qui est située en aval de la seconde vanne de contrôle 123.

L'oxygène poursuit alors sa route dans la ligne principale d'O₂ 121 en traversant un second régulateur de pression 124 pour réduire ou contrôler la pression du flux d'oxygène, par exemple pour obtenir une pression de 1,6 bar abs relatif. Le débit du flux d'O₂ peut ensuite être ajusté, par exemple entre 5 et 20 L/min, par des moyens de réglage de débit d'O₂ 125, tel un disque rotatif à orifices calibrés ou analogue, agencé sur la ligne principale d'O₂ 121 en aval du second régulateur de pression 124.

La sélection du débit d'O₂ désiré peut être opérée par l'utilisateur via un moyen de sélection de débit 196, tel un bouton rotatif ou analogue, porté par le boitier 199 du dispositif (ou selon un autre mode de réalisation, une sélection d'un débit d'O₂ via une touche ou analogue affichée sur un écran d'affichage 950), lequel moyen de sélection de débit 196 coopère avec les moyens de réglage de débit d'O₂ 125.

La vérification du débit d'oxygène peut se faire ensuite via un dispositif indicateur 126 de débit d'O₂, tel un rotamètre à bille ou analogue, agencé en aval du dispositif ou moyens de réglage de débit d'O₂ 125. Le flux d'oxygène obtenu peut alors se mélanger au flux de NO/N₂ à partir du site de jonction 130 où la ligne principale d'O₂ 121 vient se raccorder à la ligne de NO de secours 111, comme déjà mentionné.

Ce circuit de secours 110, 120 étant entièrement pneumatique, afin de contrôler la circulation de NO/N₂ au sein de la ligne de NO de secours 111, on prévoit un second tronçon de conduit 121-2 venant se raccorder fluidiquement à la ligne principale d'O₂ 121 entre la seconde vanne de contrôle 123 et le second régulateur de pression 124. Ce second tronçon de conduit 121-2 pilote la vanne pneumatique 113 du circuit de NO de secours 110 grâce à la pression d'oxygène qu'il renferme.

Plus précisément, tant que la seconde vanne de contrôle 123 est fermée, la pression du flux d'oxygène ne s'exerce pas dans le second tronçon de conduit 121-2, donc la vanne pneumatique 113 du circuit de NO 110 reste elle aussi fermée.

A l'inverse, lorsque la seconde vanne de contrôle 123 s'ouvre après actionnement par l'utilisateur du moyen d'actionnement 195 et ouverture de la première vanne de contrôle 122, comme expliqué ci-avant, le flux d'oxygène sous pression va pouvoir traverser la seconde vanne de contrôle 123, puis se répandre en aval de celle-ci, en particulier dans le second tronçon de conduit 121-2 pour alors agir pneumatiquement sur la vanne pneumatique 113 du circuit de NO 110 et l'ouvrir en libérant ainsi le passage du mélange NO/N₂ et sa circulation dans la partie aval de la ligne de NO de secours 111 située en aval de la vanne pneumatique 113.

La première vanne de contrôle 122, la seconde vanne de contrôle 123 et la vanne pneumatique 113 sont par exemple des vannes pneumatiques pilotées du type à clapet et ressort.

Autrement dit, l'activation du moyen d'actionnement 195 par l'utilisateur conduit à une libération quasi-synchronisée et/ou quasi-simultanée des flux de mélange NO/N₂ et d'O₂ au sein du circuit de NO de secours 110 et du circuit d'oxygène 120 de secours, conduisant ainsi à leur mélange à partir et en aval du site de jonction 130, c'est-à-dire dans la ligne commune de secours 140 qui débouche au niveau d'une sortie de secours 141, i.e. une sortie secondaire, par exemple portée par un connecteur de raccordement ou analogue, auquel peut être raccordé à un insufflateur de gaz manuel, c'est-à-dire un ballon de ventilation manuel ou analogue.

On comprend que le fonctionnement de ce circuit de gaz de secours ou secondaire 110, 120, est entièrement pneumatique puisqu'il ne met en œuvre que des conduits de gaz et des vannes pneumatiques pour contrôler les flux gaz.

Toutefois, comme déjà expliqué, un tel circuit entièrement pneumatique n'est pas idéal car la concentration en NO du mélange gazeux obtenu (i.e. NO/N₂ + O₂) à partir du site de jonction 130 et donc dans la ligne commune 140 de secours située en aval, varie en fonction du débit d'oxygène réglé par l'utilisateur puisque le débit de NO est fixe alors que celui d'oxygène est ajustable.

Ainsi, pour un mélange gazeux NO/N₂ à 800 ppmv de NO (reste azote), la concentration de NO varie entre 8 et 32 ppmv pour un débit d'oxygène compris entre 5 et 20 L/min, alors que pour un mélange NO/N₂ à 450 ppmv de NO, la concentration de NO varie entre 4,5 et 18 ppmv pour le même débit d'oxygène.

Or, avoir une concentration en NO qui varie en fonction du débit d'O₂ choisi, n'est pas acceptable car on doit pouvoir délivrer au patient une posologie donnée, c'est-à-dire une quantité de NO fixe correspondant à une concentration efficace pour traiter sa pathologie, en particulier dans le cadre d'une ventilation du patient par un ballon de ventilation manuelle ou BAVU en lieu et place de la ventilation normale par le ventilateur médical 2.

Il convient dès lors de pouvoir rendre la concentration de NO du mélange gazeux NO/N₂/O₂ obtenu indépendante du débit d'oxygène, lorsque le mode secours est activé par l'utilisateur et qu'une ventilation du patient via un ballon de ventilation manuelle doit être opérée.

Pour ce faire, on prévoit dans le dispositif de fourniture de NO 1 amélioré comme illustré en Fig. 3 et Fig. 4 qui schématisent un mode de réalisation de l'architecture interne d'un dispositif de fourniture de NO 1 selon la présente invention.

Tout d'abord, le dispositif de fourniture de NO 1 de Fig. 3 comprend les éléments entièrement pneumatiques du circuit de gaz de secours 110, 120 de Fig. 2, ceux-ci portent les mêmes références et ne sont donc pas réexpliqués ci-après.

Ensuite, comme visible sur Fig. 3, le dispositif de fourniture de NO 1 de l'invention comprend aussi le circuit principal de gaz 200 interne servant, lors d'un fonctionnement normal du dispositif 1, de fournir le mélange NO/N₂, via le conduit d'injection de NO 11, à la branche inspiratoire 30 du circuit ventilatoire 3 qui est relié et alimenté par le ventilateur médical 2, en gaz respiratoire, i.e. air ou mélange N₂/O₂, comme expliqué ci-avant en lien avec la Fig. 1.

Le circuit principal de gaz 200 comprend au moins une portion de conduit amont 201, à savoir ici deux portions de conduit amont 201 auxquelles viennent se raccorder fluidiquement les tronçons de canalisation d'entrée 111-1, 111-2, en un site de raccordement 205 situé entre le filtre 115 et le clapet anti-retour 116 de chaque tronçon de canalisation d'entrée 111-1, 111-2, comme représenté sur Fig. 3. Bien entendu, si seul un tronçon de canalisation d'entrée 111-1 est prévu, selon le mode de réalisation considéré, comme expliqué ci-dessus, alors seule une portion de conduit amont 201 est nécessaire.

La ou les portions de conduit amont 201 permettent d'acheminer le mélange NO/N₂ provenant des tronçons de canalisation d'entrée 111-1, 111-2 jusqu'à des moyens de contrôle de débit de NO/N₂ 202 permettant de contrôler le débit du flux de mélange NO/N₂ qui doit être ensuite fourni au conduit d'injection 11. Un mode de réalisation des moyens de contrôle de débit de NO/N₂ 202 est détaillé en Fig. 4.

Après son passage dans les moyens de contrôle de débit de NO/N₂ 202, le flux gazeux est convoyé par une portion de conduit aval 203 de la ligne principale de NO 201, 203 qui l'amène et le fournit au conduit d'injection 11, via l'orifice de sortie principal 210 qui se trouve à l'extrémité de sortie de la portion de conduit aval 203 du circuit principal de gaz 200.

Une première électrovanne 204 à plusieurs voies, ici à 3 voies, est agencée sur la portion de conduit aval 203 de la ligne principale de NO 201, 203 entre les moyens de contrôle de débit de NO/N₂ 202 et l'orifice de sortie principal 210, c'est-à-dire qu'elle est raccordée par 2 de ces 3 voies à la portion de conduit aval 203 de manière à contrôler la circulation du gaz dans la portion de conduit aval 203. Elle est par ailleurs raccordée, via sa troisième voie, à la ligne de NO de secours 111, en aval de la vanne pneumatique 113 qui est située elle-même en aval du premier régulateur de pression 112, en considérant le sens de circulation du gaz, i.e. mélange NO/N₂, dans la ligne de NO de secours 111. La première électrovanne 204 coopère ici avec la ligne de NO de secours 111 via un conduit de liaison reliant la ligne de NO de secours 111 à la troisième voie de la première électrovanne 204.

Par ailleurs, comme on le voit sur Fig. 3, la ligne de NO de secours 111 comprend une seconde électrovanne 150 à 2 voies qui est normalement en position ouverte, c'est-à-dire qu'elle laisse circuler le gaz au sein de la ligne de NO de secours 111, et le dispositif à orifice calibré 114 ou analogue permettant de réguler le débit gazeux, i.e. du mélange NO/N₂, qui sur Fig. 2 est agencé à proximité du dispositif indicateur de débit de NO 117. La seconde électrovanne 150 se situe donc entre la vanne pneumatique 113 et la première électrovanne 204 à 3 voies.

La seconde électrovanne 150 est normalement en position ouverte mais peut être commandée par les moyens de pilotage 900, notamment en cas d'activation du mode secours en cas de défaillance légère, comme expliqué ci-après, ou en cas de besoin de devoir ventiler un patient à l'aide de l'insufflateur manuel pour réaliser des manœuvres de recrutement alvéolaire par exemple.

Selon un autre mode de réalisation (non schématisé), la première électrovanne 204 à 3 voies et la seconde électrovanne 150 à 2 voies peuvent être remplacées par une électrovanne unique à 5 voies qui serait installée en lieu et place de la première électrovanne 204 à 3 voies (la seconde électrovanne 150 serait alors supprimée).

En outre, le dispositif de fourniture de NO 1 de l'invention comprend enfin des moyens de mesure de débit d'oxygène 160 agencés sur la ligne principale d'O₂ 121 entre le second régulateur de pression 124 et le dispositif ou les moyens de réglage de débit d'O₂ 125.

Ces moyens de mesures de débit d'oxygène 160 peuvent comprendre un capteur de débit ou un capteur de pression différentiel dont les prises de pression 161, 162 sont raccordées en amont et en aval d'une restriction de flux 163, tel un système à venturi, un orifice calibré ou autre, afin de mesurer un différentiel de pression (ΔP), i.e. une perte de charge, permettant d'en déduire un débit d'oxygène (Q_{O2}).

Fig. 4 représente un mode de réalisation détaillé des moyens de contrôle de débit de NO/N₂ 202 servant à contrôler le débit de mélange NO/N₂ au sein du circuit principal de gaz 200 agencé dans le boitier 199 du dispositif de fourniture de NO 1.

Comme on le voit, les deux portions de conduit amont 201 du circuit principal de gaz 200 se rejoignent pour former un tronçon commun 201-1 véhiculant le mélange NO/N₂. Chaque portion de conduit amont 201 comprend une première valve de contrôle 700 agencée en amont du site de jonction des deux portions de conduit amont 201, laquelle sert à autoriser ou stopper la circulation du flux de mélange NO/N₂ amené par l'un ou l'autre des portions de conduit amont 201.

Le tronçon commun 201-1 se divise ensuite en deux tronçons secondaires 201-2 agencés en parallèle l'un de l'autre, qui comprennent chacun un dispositif régulateur de pression 701 et un capteur de pression 702.

Les deux tronçons secondaires 201-2 se divisent à leur tour en plusieurs sous-tronçons 201-3 qui sont eux aussi agencés en parallèle les uns des autres, à savoir ici deux sous-tronçons 201-3. Chaque sous-tronçons 201-3 comprend une seconde valve de contrôle 703. Des sous-tronçons 201-3 comprennent aussi des moyens de contrôle de débit additionnels 704, par exemple un (des) orifice calibré ou analogue, permettant d'ajuster le débit à une valeur de débit souhaitée.

Bien entendu, les moyens de contrôle de débit de NO/N₂ 202 peuvent comprendre davantage de sous-tronçons 201-3 notamment équipés chacun d'une valve de contrôle 703 et de moyens de contrôle de débit aditionnels 704, typiquement un orifice calibré, afin de permettre de délivrer une plus grande variété de débits de NO, si nécessaire. De préférence, la (les) valve de contrôle 703 est une valve de type tout ou rien (TOR). Le débit de gaz qui y circule est fixe et dépend de la pression du régulateur de pression 701, i.e. un détendeur, et de la section de l'orifice calibré.

En fait, les moyens de pilotage 900 du dispositif de fourniture de NO 1, tel une carte électronique portant un ou des microprocesseurs mettant en œuvre un ou des algorithmes, pilotent les premières valves de contrôle 700 et les secondes valves de contrôle 703 afin de diriger les gaz dans les tronçons adéquats permettant d'obtenir le dosage de NO souhaité.

La teneur en NO souhaitée est choisie par l'utilisateur via des moyens de sélection de teneur en NO (non schématisés) agencés sur le dispositif, par exemple une ou des touches, curseurs, boutons de sélection, notamment un bouton rotatif, ou analogues, lesquels permettent de sélectionner ou fixer une teneur de NO désirée.

Avantageusement, le dispositif de fourniture de NO 1 de l'invention est équipé d'un écran d'affichage 950 d'informations, typiquement un écran numérique, préférentiellement un écran tactile, en couleurs ou en noir et blanc, configuré pour afficher la valeur de de teneur en NO désirée et/ou d'autres informations, comme la teneur en NO dans les bouteilles de NO 5 ou encore les valeurs de débit de NO ou d'O₂, et par exemple aussi la concentration en NO₂, le débit délivré par le ventilateur...

Selon un mode de réalisation, l'écran d'affichage 950 d'informations est un écran numérique à commande tactile et les moyens de sélection de teneur en NO sont des touches de sélection à actionnement tactile, affichées sur l'écran d'affichage 950 numérique tactile. La sélection ou la fixation de la concentration en NO désirée se fait donc par appui digital de la part de l'utilisateur sur la ou les touches de sélection à actionnement tactile affichées sur l'écran d'affichage 950.

Une fois la teneur en NO sélectionnée, les moyens de pilotage 900 du dispositif de fourniture de NO 1 calculent le débit de mélange NO/N₂ à fournir, notamment en fonction d'autres paramètres, tel que la teneur en NO du mélange NO/N₂ provenant des bouteilles 5, et aussi le débit de gaz respiratoire (typiquement air ou air/O₂) fourni par le ventilateur 2, et déterminent ensuite quelles valves de contrôle 700, 703 doivent être ouvertes ou fermées afin de diriger les gaz dans les tronçons adaptés à l'obtention du dosage de NO fixé.

Les sous-tronçons 201-3 se réunissent ensuite, en amont de la première électrovanne 204 à 3 voies, en une ligne unique qui forme la portion de conduit aval 203 du circuit principal de gaz 200. Elle peut comprendre un capteur de pression 705 ou analogue, en amont de la première électrovanne 204, afin de pouvoir corriger éventuellement le débit qui dépend de la variation de pression (ΔP) du gaz lorsqu'il traverse les moyens de contrôle de débit additionnels 704, typiquement un ou des orifices calibrés.

De plus, on voit aussi que le dispositif de fourniture de NO 1 de l'invention comprend en outre une ligne de purge 800 communiquant avec l'extérieur via un orifice de purge 802 agencé dans le boitier 199. La ligne de purge 800 se ramifie en deux tronçons de purge 801 venant se raccorder aux deux portions de conduit amont 201 afin de pouvoir opérer une purge gazeuse de ces deux portions de conduit amont 201. Chaque tronçon de purge 801 comprend une vanne de purge 803 pilotée par les moyens de pilotage 900 de manière à pouvoir éliminer toutes les espèces NO₂ pouvant se former dans le gaz résiduel présent dans les canalisations par oxydation des molécules de NO par de l'oxygène, lorsque le dispositif 1 n'est pas en fonctionnement, c'est-à-dire n'est pas utilisé.

En fonctionnement normal, c'est-à-dire lorsque l'utilisateur n'a pas actionné le moyen d'actionnement 195, le mélange NO/N₂ chemine dans le circuit principal de gaz 200 du dispositif de fourniture de NO 1 de l'invention, via les moyens de contrôle de débit de NO/N₂ 202, depuis l'un des ports d'entrée de gaz 101 jusqu'au port de sortie principal 210 qui fournit le mélange NO/N₂ au conduit d'injection de NO 11 de manière à pouvoir ensuite l'injecter à la concentration en NO désirée, dans la branche inspiratoire 30 du circuit ventilatoire 3 relié au ventilateur médical 2, comme expliqué ci-avant en lien avec Fig. 1 et Fig. 4.

Dans ce cas, la seconde électrovanne 150 à 2 voies est en position « normalement ouverte », c'est-à-dire qu'elle n'interrompt pas le passage de gaz dans la ligne de NO de secours 111, et la première électrovanne 204 à 3 voies est en position de « fonctionnement normal » autorisant le passage du flux gazeux dans la portion de conduit aval 203 jusqu'à l'orifice de sortie principal 210.

La première électrovanne 204 est commandée par les moyens de pilotage 900 pour autoriser ou stopper tout passage de mélange NO/N₂ provenant des moyens de contrôle (i.e. de réglage) de débit de NO 202.

En cas de défaillance grave ou sévère du dispositif de fourniture de NO 1 de l'invention conduisant à une perte d'énergie électrique et/ou à un arrêt de fonctionnement des moyens de pilotage 900 du dispositif 1, voire du ventilateur médical 2, notamment d'un logiciel ou autre programme ou algorithme de pilotage mis en œuvre par un ou des microprocesseurs desdits moyens de pilotage, la seconde électrovanne 150 à 2 voies reste en position « normalement ouverte » et la première électrovanne 204 à 3 voies reste en position de « fonctionnement normal » de manière à autoriser une circulation du flux gazeux dans la portion de conduit aval 203 jusqu'à l'orifice de sortie principal 210. Ceci permet, après actionnement par l'utilisateur du moyen d'actionnement 195, un passage en mode secours de type entièrement pneumatique, comme expliqué en lien avec Fig. 2. Il en résulte donc la délivrance par la ligne de NO de secours 111, en aval du dispositif indicateur de débit de NO 117, d'un flux de NO/N₂ a un débit fixe de l'ordre de 230 mL/min par exemple qui se mélange (en 130) au flux d'oxygène à débit ajustable provenant de la ligne principale d'O₂ 121.

Le mélange gazeux de secours formé de NO/N₂/O₂ ainsi obtenu peut être alors convoyé par la ligne commune 140 jusqu'à la sortie secondaire ou de secours 141, qui fournit alors ce mélange gazeux de secours à un ballon de ventilation manuel, aussi appelé insufflateur manuel ou BAVU. Ensuite, la ventilation du patient se fait alors via le ballon de ventilation manuelle pour assurer une fourniture de NO au patient malgré la panne sévère ou analogue.

Par contre, si la défaillance du dispositif de fourniture de NO 1 de l'invention est une défaillance légère ne conduisant pas à une perte totale d'énergie électrique et/ou à un arrêt de fonctionnement des moyens de pilotage 900 du dispositif 1 et/ou si le personnel soignant décide d'opérer une ventilation du patient via un ballon de ventilation manuelle tout en souhaitant respecter la posologie en NO, alors le dispositif de fourniture de NO 1 de l'invention et plus particulièrement les moyens de pilotage 900 et l'écran d'affichage 950 restent alimentés en énergie électrique par les moyens de fourniture d'énergie électrique, donc les moyens de pilotage 900 restent aptes à fonctionner.

Dans ce cas, l'utilisateur désirant passer le dispositif 1 en mode secours ou de ventilation par BAVU, pour assurer une fourniture de gaz au patient via un ballon de ventilation manuel ou BAVU, va actionner, comme précédemment, le moyen d'actionnement 195, ce qui va alors engendrer une ouverture de la première vanne de contrôle 122 en autorisant alors la circulation du flux d'oxygène dans la ligne principale d'O₂ 121 jusqu'à la seconde vanne de contrôle 123 qui va alors aussi s'ouvrir et laisser passer l'oxygène dans la partie aval du circuit d'oxygène 120, située en aval de la seconde vanne de contrôle 123, comme expliqué ci-dessus en lien avec Fig. 2.

Les moyens de mesure de débit d'oxygène 160 qui sont agencés sur la ligne principale d'O₂ 121 en aval du second régulateur de pression 124 vont alors pouvoir mesurer le débit d'oxygène (Q_{O2}) et transmettre cette mesure de débit d'O₂ (i.e. signal ou valeur) aux moyens de pilotage 900 afin de permettre de calculer le débit de NO/N₂ à fournir comme expliqué ci-après.

Le flux d'oxygène peut ensuite poursuivre son cheminement jusqu'au site de jonction 130 où s'opère le mélange O₂/NO/N₂, via les moyens de réglage de débit d'O₂ 125 et le dispositif indicateur de débit d'O₂ 126, tel un rotamètre à bille ou analogue, comme déjà expliqué.

Toutefois, dans ce cas, le moyen d'actionnement 195 est relié électriquement aux moyens de pilotage 900 et configuré pour leur fournir une information d'actionnement, à savoir un signal d'activation correspondant à la position du moyen d'actionnement 195, à savoir en mode secours (i.e. secours activé) ou en mode normal (i.e. secours non activé). Ce signal d'activation est fourni électriquement aux moyens de pilotage 900 du dispositif de fourniture de NO 1 de l'invention, qui le reçoivent et le traitent pour déterminer si le moyen d'actionnement 195 a été actionné par l'utilisateur afin de déclencher le mode de secours ou de ventilation par BAV, en vue de réaliser une ventilation du patient via un ballon de ventilation manuelle raccordé à la sortie secondaire, i.e. la sortie de secours 141, dispositif de fourniture de NO 1 de l'invention, par exemple via un tuyau flexible.

Si tel est le cas, les moyens de pilotage 900 du dispositif de fourniture de NO 1 de l'invention qui restent alimentés en énergie électrique (i.e. courant électrique), rétroagissent, en réponse à ce signal, sur les moyens de contrôle de débit de NO/N₂ 202 pour fournir le mélange NO/N₂ au débit souhaité et sur la première électrovanne 204 à 3 voies pour autoriser le passage du flux de NO/N₂ de la portion de conduit aval 203 du circuit principal de gaz 200 à la partie aval de la ligne de NO de secours 111 située en aval de la première électrovanne 204 et acheminant le flux de NO/N₂ via le dispositif indicateur de débit de NO 117, jusqu'au site de mélange 130 où se rejoignent la ligne principale d'O₂ 121 et la ligne de NO de secours 111, comme déjà expliqué.

Ainsi, le mélange NO/N₂ étant réalisé au sein des moyens de contrôle de débit de NO/N₂ 202, il est possible de fixer le débit de NO le plus adapté. Ce débit de mélange NO/N₂ à fournir par les moyens de contrôle de débit de NO/N₂ 202 peut être calculé par les moyens de pilotage 900 du dispositif de fourniture de NO 1 de l'invention à partir de la teneur en NO finale souhaitée, de la composition du mélange NO/N₂ dans les bouteilles 5 et du débit d'oxygène mesuré par les moyens de mesure de débit d'oxygène 160 qui sont agencés sur la ligne principale d'O₂ 121 en aval du second régulateur de pression 124.

La teneur en NO finale souhaitée est donc, dans ce cas, choisie par l'utilisateur via les moyens de réglage de teneur en NO agencés sur le dispositif, de préférence une ou des touches numériques à actionnement digital affichées sur l'écran d'affichage numérique 950 qui reste lui aussi alimenté en courant électrique.

Pouvoir utiliser un mélange NO/N₂ réalisé au sein des moyens de contrôle de débit de NO/N₂ 202, en cas d'activation du circuit de secours et en l'absence d'un défaut complet du dispositif 1, c'est-à-dire en cas de défaillance légère, et/ou de désir de réaliser une ventilation du patient via un ballon de ventilation manuelle, présente l'avantage de garantir une plus grande précision de concentration en NO du mélange de secours fourni au patient étant donné que la dite teneur en NO finale, peut être réglée par l'utilisateur via les moyens de réglage de teneur en NO (i.e. choix ou sélection) et que les moyens de pilotage 900 utilisent cette valeur de teneur en NO réglée, la concentration du NO dans le mélange NO/N₂ fourni par les bouteilles de NO 5 et la valeur de débit d'O₂ mesurée pour déterminer le débit de NO/N₂ à fournir et agir en conséquence sur les moyens de contrôle de débit de NO/N₂ 202, notamment les premières valves de contrôle 700 et les secondes valves de contrôle 703, pour fournir le débit de NO/N₂ adéquat en contrôlant le passage du gaz notamment dans les tronçons secondaires 201-2, les dispositif régulateur de pression 701, les deux tronçons secondaires 201-2 et les sous-tronçons 201-3 comprenant les moyens de contrôle de débit additionnels 704, par exemple des orifice calibrés ou analogues, permettant d'ajuster le débit à une valeur de débit souhaitée.

Autrement dit, grâce à l'incorporation des moyens de mesure de débit d'oxygène 160 sur la ligne principale d'O₂ 121, en aval du second régulateur de pression 124, qui mesurent le débit d'oxygène (Q_{O2}) et transmettent cette mesure de débit d'O₂ aux moyens de pilotage, ces derniers peuvent calculer une consigne de débit en NO/N₂ à fournir par les moyens de contrôle de débit de NO/N₂ 202 en prenant en compte par ailleurs non seulement la consigne de NO réglée par l'utilisateur mais aussi la concentration du NO dans le mélange NO/N₂ provenant des bouteilles de NO 5.

Ceci conduit à pouvoir obtenir à partir du site de mélange 130, un mélange final de teneur précise en NO puisque le débit de mélange NO/N₂ n'est plus fixe, (e.g. égale à 230 mL/min) mais est ajustable en fonction des autres paramètres ayant une influence.

Toutefois, dans ce cas, il est indispensable d'éviter que le flux d'oxygène qui traverse la seconde vanne de contrôle 123 et qui agit sur la vanne pneumatique 113, ne soit à l'origine d'un apport excessif de NO via la ligne de NO de secours 111 au travers de la seconde électrovanne 150 à 2 voies. Pour ce faire, lorsqu'ils reçoivent le signal d'activation provenant du moyen d'actionnement 195, lequel correspond à un passage en mode secours, les moyens de pilotage 900 du dispositif de fourniture de NO 1 de l'invention sont aussi configurés pour agir aussi sur la seconde électrovanne 150 à 2 voies, qui est normalement en position ouverte, pour la fermer et ainsi empêcher que le mélange NO/N₂ ne puisse circuler au sein de la ligne de NO de secours 111 et alimenter la partie aval de la ligne de NO de secours 111 située en aval de la première électrovanne 204.

Le mélange NO/N₂/O₂ ainsi obtenu est alors, comme précédemment, acheminé par la ligne commune 140 qui débouche au niveau d'une sortie de secours 141, i.e. sortie secondaire, pour fournir le mélange gazeux de secours formé de NO/N₂/O₂ et pouvoir l'amener ensuite, via un conduit flexible ou analogue, jusqu'à un ballon de ventilation manuelle pour opérer une ventilation manuelle du patient avec le mélange gazeux NO/N₂/O₂ ayant la posologie désirée

A l'inverse, en cas de défaillance grave du dispositif 1 avec défaut d'alimentation électrique, aucun signal d'activation n'est généré par le moyen d'actionnement 195 et/ou n'est utilisé/traité par les moyens de pilotage 900 et/ou l'écran d'affichage ne peut pas fonctionner, donc en cas d'activation du mode secours via le moyen d'actionnement 195, le dispositif 1 repasse en mode secours entièrement pneumatique, comme expliqué en lien avec Fig. 2.

Le dispositif de fourniture de NO 1 selon l'invention présente un niveau de sécurité plus élevé en permettant d'ajuster plus précisément la teneur en NO en cas de défaillance ou de panne légère, sans perte d'alimentation électrique, ou lorsque l'utilisateur veut passer en ventilation manuelle du patient par BAVU.

D'une façon générale, le dispositif de fourniture de NO 1 selon l'invention est particulièrement adapté à une utilisation au sein d'une installation 100 d'administration de gaz thérapeutique à base de NO (< 100 ppmv), d'O₂ (>21% vol) et d'azote à un patient P, telle l'installation 100 de Fig. 1, mise en oeuvre pour traiter un ou des patients souffrant d'une pathologie ou d'une condition médicale engendrant une hypertension artérielle pulmonaire aiguë, en particulier les vasoconstrictions pulmonaires chez l'adulte ou l'enfant, y compris les nouveau-nés, par exemple pour traiter une PPHN chez un nouveau-né, ou une hypertension pulmonaire chez une personne subissant une chirurgie cardiaque.

Autrement dit, selon un autre aspect (non-revendiqué), la présente divulgation concerne donc aussi une méthode de traitement d'une personne, appelée un patient, souffrant d'une pathologie ou d'une condition médicale engendrant une hypertension artérielle pulmonaire aiguë, en particulier des vasoconstrictions pulmonaires chez l'adulte ou l'enfant, y compris les nouveau-nés, par exemple pour traiter une hypertension pulmonaire persistante du nouveau-né (PPHN) chez un nouveau-né, bébé ou analogue, ou une hypertension pulmonaire chez une personne subissant une chirurgie cardiaque, dans laquelle on met en oeuvre une administration par inhalation audit patient en ayant besoin, d'un mélange gazeux contenant de l'oxygène (de préférence >21%vol), de l'azote et du NO (de préférence < 100 ppmv), ledit mélange gazeux étant fourni par une installation 100 d'administration de gaz thérapeutique selon l'invention comprenant un dispositif de fourniture de NO 1 selon l'invention, lequel dispositif de fourniture de NO 1 est alimenté en mélange NO/N₂ par au moins un récipient de gaz sous pression 5, de préférence plusieurs récipients de gaz sous pression 5, telles des bouteilles de NO/N₂, et en oxygène par au moins un récipient d'oxygène sous pression 52, telle une bouteille d'O₂, et lequel dispositif de fourniture de NO 1 étant apte à et conçu pour fournir : soit un mélange NO/N₂ via l'orifice de sortie principal 210, au circuit de gaz respiratoire 3 raccordé à un ventilateur médical 2 de l'installation 100 d'administration de gaz thérapeutique, soit un mélange NO/N₂/O₂ via l'orifice secondaire 141, à un insufflateur manuel, en particulier un ballon d'insufflation manuel ou BAVU.

D'une façon générale, dans le cadre de la présente invention, tous les termes « moyens de » ou « moyen de » sont considérés comme totalement équivalents et substituables par les termes « dispositif de », par exemple les termes « moyens de pilotage » peuvent être remplacés par « dispositif de pilotage », les termes « moyens de mesure » peuvent être remplacés par « dispositif de mesure ».....

## Revendications

1. Dispositif de fourniture de NO (1) comprenant :
- un circuit principal de gaz (200) comprenant au moins une ligne principale de NO (201, 203) reliant fluidiquement au moins un port d'entrée de NO (101) à un orifice de sortie principal (210) pour acheminer un mélange NO/N₂ dudit au moins un port d'entrée de NO (101) audit orifice de sortie principal (210), et
- un circuit de secours (110, 120) comprenant un circuit de NO de secours (110) et un circuit d'O₂ de secours (120), dans lequel :
. le circuit de NO de secours (110) comprend une ligne de NO de secours (111) alimentée en mélange NO/N₂ provenant dudit au moins un port d'entrée de NO (101), et
. le circuit d'O₂ de secours (120) comprend une ligne principale d'O₂ (121) en communication fluidique avec un port d'entrée d'O₂ (102), la ligne principale d'O₂ (121) venant se raccorder fluidiquement (130) à la ligne de NO de secours (111) pour former une ligne commune (140) en communication fluidique avec une sortie secondaire (141) fournissant un mélange NO/N₂/O₂, et dans lequel :
- la ligne principale de NO (201, 203) comprend des moyens de contrôle de débit de NO/N₂ (202) pilotés par des moyens de pilotage (900), et **caractérisé en ce que** :
- la ligne principale de NO (201, 203) est raccordée fluidiquement à la ligne de NO de secours (111) en aval des moyens de contrôle de débit de NO/N₂, par l'intermédiaire d'une première électrovanne (204) à plusieurs voies, et
- il comprend en outre un écran numérique (950) d'affichage d'informations à commande tactile configuré pour afficher au moins une touche de sélection tactile,
- ladite au moins une touche de sélection tactile affichée sur l'écran d'affichage (950) est configurée pour permettre à l'utilisateur de fixer ou sélectionner une teneur en NO désirée comprise entre 1 et 80 ppmv, et
- ledit écran numérique (950) est configuré pour afficher en outre la teneur en NO désirée.

2. Dispositif selon la revendication 1, **caractérisé en ce que** les touches de sélection tactile permettent à l'utilisateur d'opérer un choix parmi plusieurs teneurs en NO proposées.

3. Dispositif selon la revendication 1, **caractérisé en ce que** les touches de sélection tactile comprennent des touches « + » et « - » permettant d'incrémenter ou de décrémenter une valeur de NO donnée par palier.

4. Dispositif selon la revendication 3, **caractérisé en ce que** les touches de sélection tactile permettent d'incrémenter ou de décrémenter la valeur de NO donnée de 1 ppmv en 1 ppmv.

5. Dispositif selon l'une des revendications 1 à 4, **caractérisé en ce que** ladite teneur en NO désirée qui est choisie ou sélectionnée par l'utilisateur par action sur ladite au moins une touche de sélection tactile, est fournie aux moyens de pilotage (900) du dispositif (1) de fourniture de NO.

6. Dispositif selon la revendication 1, **caractérisé en ce que** l'écran d'affichage est à affichage en couleurs.

7. Dispositif selon la revendication 1, **caractérisé en ce que** l'écran d'affichage est configuré pour afficher en outre une teneur en NO dans les bouteilles de NO (5), les valeurs de débit de NO ou d'O₂, la concentration en NO₂ et/ou le débit délivré par un ventilateur.

8. Dispositif selon la revendication 1, **caractérisé en ce que** la première électrovanne (204) est à au moins 3 voies, de préférence à 3 voies.

9. Dispositif selon les revendications 1 et 5, **caractérisé en ce que** la première électrovanne (204) à plusieurs voies est pilotée par les moyens de pilotage (900) de manière à diriger le flux de mélange NO/N₂ provenant des moyens de contrôle de débit de NO/N₂ (202):
- soit dans une portion aval de la ligne de NO de secours (111) en communication fluidique avec la ligne commune (140) en communication fluidique avec la sortie secondaire (141),
- soit dans une portion aval (203) de la ligne principale de NO (201, 203) en communication fluidique avec l'orifice de sortie principal (210).

10. Dispositif selon les revendication 1 et 5, **caractérisé en ce que** :
- il comprend des moyens de réglage de teneur en NO configurés pour permettre à un utilisateur de choisir une teneur en NO désirée et fournir ladite teneur en NO désirée aux moyens de pilotage (900), les moyens de réglage de teneur en NO comprenant ladite au moins une touche de sélection tactile affichée sur l'écran d'affichage (950) et
- les moyens de pilotage (900) sont configurés pour calculer un débit de mélange NO/N₂ à fournir correspondant à la teneur en NO désirée et piloter les moyens de contrôle de débit de NO/N₂ (202) pour fournir ledit débit de mélange NO/N₂ calculé.

11. Dispositif selon la revendication 1, **caractérisé en ce que** la sortie secondaire (141) est configurée pour être raccordée fluidiquement à un insufflateur manuel, en particulier un ballon de ventilation manuelle (BAVU), de préférence via un conduit flexible de raccordement fluidique.

12. Dispositif selon la revendication 5, **caractérisé en ce que** les moyens de pilotage (900) comprennent au moins un microprocesseur.

13. Installation (100) d'administration de gaz thérapeutique contenant du NO, à un patient (P) comprenant un dispositif de fourniture de NO (1) selon l'une des revendications précédentes, alimenté en mélange NO/N₂ par au moins un récipient de gaz sous pression (5) et en oxygène par un récipient d'oxygène sous pression (52), ledit dispositif de fourniture de NO (1) fournissant :
- soit un mélange NO/N₂ via l'orifice de sortie principal (210), à un circuit de gaz respiratoire (3) raccordé à un ventilateur médical (2),
- soit un mélange NO/N₂/O₂ via l'orifice secondaire (141), à un insufflateur manuel ou BAVU.

## Patentansprüche

1. NO-Zufuhrvorrichtung (1), umfassend:
- einen Hauptgaskreislauf (200) mit mindestens einer NO-Hauptleitung (201, 203), die mindestens einen NO-Einlassanschluss (101) mit einem Hauptauslass (210) fluidisch verbindet, um ein NO/N₂ von dem mindestens einen NO-Einlassanschluss (101) zu dem Hauptauslassanschluss (210) zu leiten, und
- einen Notkreislauf (110, 120) mit einem NO-Notkreislauf (110) und einem O₂-Notkreislauf (120), wobei:
. der NO-Notkreislauf (110) eine NO-Notleitung (111) umfasst, die mit einem NO/N₂-Gemisch aus dem mindestens einen NO-Eingangsanschluss (101) versorgt wird, und
. der O₂-Notfallkreislauf (120) eine O₂-Hauptleitung (121) umfasst, die in Fluidverbindung mit einem O₂-Eingangsanschluss (102) steht, wobei die O₂-Hauptleitung₂ (121) fluidisch (130) mit der NO-Notfallleitung (111) verbunden ist, um eine gemeinsame Leitung (140) in fluidischer Verbindung mit einem sekundären Ausgang (141) zu bilden, der ein NO/N₂/O₂ -Gemisch liefert,
wobei:
- die NO-Hauptleitung (201, 203) eine NO/N₂-Durchflusssteuereinrichtung (202) umfasst, die von einer Steuereinrichtung (900) gesteuert wird,
**dadurch gekennzeichnet, dass**:
- die NO-Hauptleitung (201, 203) stromabwärts der NO/N₂-Durchflusssteuereinrichtung über ein erstes Mehrweg-Magnetventil (204) fluidisch mit der NO-Notleitung (111) verbunden ist, und
- es umfasst außerdem einen digitalen Bildschirm (950) zur Anzeige von Informationen mit Touch-Steuerung, der so konfiguriert ist, dass er mindestens eine Touch-Auswahltaste anzeigt,
- wobei die mindestens eine auf dem Bildschirm (950) angezeigte Touch-Auswahltaste so konfiguriert ist, dass der Benutzer einen gewünschten NO-Gehalt zwischen 1 und 80 ppmv festlegen oder auswählen kann, und
- wobei der digitale Bildschirm (950) so konfiguriert ist, dass er zusätzlich den gewünschten NO-Gehalt anzeigt.

2. Vorrichtung nach Anspruch 1, **dadurch gekennzeichnet, dass** die Touch-Auswahltasten es dem Benutzer ermöglichen, eine Auswahl aus mehreren vorgeschlagenen NO-Gehalten zu treffen.

3. Vorrichtung nach Anspruch 1, **dadurch gekennzeichnet, dass** die Touch-Auswahltasten die Tasten "+" und "-" umfassen, mit denen ein bestimmter NO-Wert schrittweise erhöht oder verringert werden kann.

4. Vorrichtung nach Anspruch 3, **dadurch gekennzeichnet, dass** die Touch-Auswahltasten es ermöglichen, den gegebenen NO-Wert um 1 ppmv in 1 ppmv zu erhöhen oder zu verringern.

5. Vorrichtung nach einem der Ansprüche 1 bis 4, **dadurch gekennzeichnet, dass** der vom Benutzer durch Betätigen der mindestens einen Touch-Auswahltaste gewählte oder ausgewählte gewünschte NO-Gehalt an die Steuereinrichtung (900) der NO-Zuführvorrichtung (1) weitergeleitet wird.

6. Vorrichtung nach Anspruch 1, **dadurch gekennzeichnet, dass** der Bildschirm eine Farbdarstellung aufweist.

7. Vorrichtung nach Anspruch 1, **dadurch gekennzeichnet, dass** der Bildschirm so konfiguriert ist, dass er zusätzlich den NO-Gehalt in den NO-Flaschen (5), die Durchflusswerte von NO oder O₂, die NO₂-Konzentration und/oder den von einem Ventilator gelieferten Durchfluss anzeigt.

8. Vorrichtung nach Anspruch 1, **dadurch gekennzeichnet, dass** das erste Magnetventil (204) mindestens 3-Wege-, vorzugsweise 3-Wege-Magnetventil ist.

9. Vorrichtung gemäß den Ansprüchen 1 und 5, **dadurch gekennzeichnet, dass** das erste Mehrweg-Magnetventil (204) durch die Steuermittel (900) so gesteuert wird, dass der NO/N₂-Mischstrom,der von den NO/N₂-Durchflusssteuermitteln (202) kommt,
- entweder in einen stromabwärtigen Abschnitt der NO-Reserveleitung (111), die in Fluidverbindung mit der gemeinsamen Leitung (140) steht, die wiederum in Fluidverbindung mit dem sekundären Auslass (141) steht,
- oder in einen stromabwärtigen Abschnitt (203) der NO-Hauptleitung (201, 203), die in Fluidverbindung mit der Hauptauslassöffnung (210) steht.

10. Vorrichtung gemäß den Ansprüchen 1 und 5, **dadurch gekennzeichnet, dass**:
- sie umfasst Mittel zur Einstellung des NO-Gehalts, die so konfiguriert sind, dass ein Benutzer einen gewünschten NO-Gehalt auswählen und diesen gewünschten NO-Gehalt an die Steuermittel (900) liefern kann, wobei die Mittel zur Einstellung des NO-Gehalts die mindestens eine auf dem Bildschirm (950) angezeigte taktile Auswahltaste umfassen und
- die Steuermittel (900) so konfiguriert sind, dass sie einen zu liefernden NO/N₂-Mischungsdurchfluss berechnen, der dem gewünschten NO-Gehalt entspricht, und die NO/N₂-Durchflusskontrollmittel (202) so steuern, dass sie den berechneten NO/N₂-Mischungsdurchfluss liefern.

11. Vorrichtung nach Anspruch 1, **dadurch gekennzeichnet, dass** der sekundäre Ausgang (141) so konfiguriert ist, dass er fluidisch mit einem manuellen Beatmungsgerät, insbesondere einem manuellen Beatmungsballon (BAVU), vorzugsweise über eine flexible Fluidverbindung, verbunden werden kann.

12. Vorrichtung nach Anspruch 5, **dadurch gekennzeichnet, dass** die Steuermittel (900) mindestens einen Mikroprozessor umfassen.

13. Vorrichtung (100) zur Verabreichung von NO-haltigem therapeutischem Gas an einen Patienten (P), umfassend eine NO-Zufuhrvorrichtung (1) gemäß einem der vorstehenden Ansprüche, die mit einem NO/N₂ aus mindestens einem Druckgasbehälter (5) und mit Sauerstoff aus einem Drucksauerstoffbehälter (52) versorgt wird, wobei die NO-Versorgungsvorrichtung (1) :
- entweder ein NO/N₂-Gemisch über die Hauptauslassöffnung (210) an einen mit einem medizinischen Beatmungsgerät (2) verbundenen Atemgaskreislauf (3)
- oder ein NO/N₂/O₂-Gemisch über die Nebenöffnung (141) an ein manuelles Beatmungsgerät oder ein BAVU.

## Claims

1. A NO supply device (1) comprising:
- a main gas circuit (200) comprising at least one NO main line (201, 203) fluidically connecting at least one NO inlet port (101) to a main outlet port (210) for conveying an NO/N₂ from said at least one NO inlet port (101) to said main outlet port (210), and
- a backup circuit (110, 120) comprising a backup NO circuit (110) and a backup O₂ circuit (120), wherein:
. the backup NO circuit (110) comprises a backup NO line (111) supplied with NO/O₂ mixture from said at least one NO inlet port (101), and
. the backup O₂ circuit (120) comprises a main O₂ line (121) in fluid communication with an O₂ inlet port (102), the main O₂ line (121) being fluidly connected (130) to the backup NO line (111) to form a common line (140) in fluidic communication with a secondary outlet (141) supplying an NO/N₂/O₂ mixture,
wherein:
- the main NO line (201, 203) comprises NO/N₂ flow control means (202) controlled by control means (900),
**characterized in that**:
- the main NO line (201, 203) is fluidly connected to the backup NO line (111) downstream of the NO/N₂ flow control means, via a first multi-way solenoid valve (204), and
- it further comprises a touch-controlled digital information display screen (950) configured to display at least one touch selection key,
- said at least one touch-sensitive selection key displayed on the display screen (950) is configured to allow the user to set or select a desired NO content between 1 and 80 ppmv, and
- said digital display (950) is configured to further display the desired NO content.

2. Device according to claim 1, **characterized in that** the touch selection keys allow the user to choose from among several proposed NO contents.

3. Device according to claim 1, **characterized in that** the touch selection keys include "+" and "-" keys for incrementing or decrementing a given NO value in steps.

4. Device according to claim 3, **characterized in that** the touch selection keys allow the given NO value to be increased or decreased by 1 ppmv at a time.

5. Device according to one of claims 1 to 4, **characterized in that** said desired NO content, which is chosen or selected by the user by pressing said at least one touch selection key, is supplied to the control means (900) of the NO supply device (1).

6. Device according to claim 1, **characterized in that** the display screen is a color display.

7. Device according to claim 1, **characterized in that** the display screen is configured to additionally display the NO content in the NO cylinders (5), the NO or O₂ flow rates, the NO₂ concentration, and/or the flow rate delivered by a ventilator.

8. Device according to claim 1, **characterized in that** the first solenoid valve (204) is at least a 3-way valve, preferably a 3-way valve.

9. Device according to claims 1 and 5, **characterized in that** the first multi-way solenoid valve (204) is controlled by the control means (900) so as to direct the flow of NO/N₂ mixture from the NO/N₂ flow control means (202):
- either into a downstream portion of the backup NO line (111) in fluid communication with the common line (140) in fluid communication with the secondary outlet (141),
- or into a downstream portion (203) of the main NO line (201, 203) in fluid communication with the main outlet port (210).

10. Device according to claims 1 and 5, **characterized in that**:
- it comprises NO content adjustment means configured to allow a user to select a desired NO content and provide said desired NO content to the control means (900), the NO content adjustment means comprising said at least one touch selection key displayed on the display screen (950) and
- the control means (900) are configured to calculate a NO/N₂ mixture flow rate to be supplied corresponding to the desired NO content and to control the NO/N₂ flow rate control means (202) to supply said calculated NO/N₂ mixture flow rate.

11. Device according to claim 1, **characterized in that** the secondary outlet (141) is configured to be fluidically connected to a manual insufflator, in particular a manual ventilation bag (BAVU), preferably via a flexible fluid connection conduit.

12. Device according to claim 5, **characterized in that** the control means (900) comprise at least one microprocessor.

13. Installation (100) for administering therapeutic gas containing NO to a patient (P), comprising an NO supply device (1) according to one of the preceding claims, supplied with a mixture of NO/N₂ by at least one pressurized gas container (5) and with oxygen by a pressurized oxygen container (52), said NO supply device (1) supplying:
- either an NO/N₂ mixture via the main outlet port (210) to a respiratory gas circuit (3) connected to a medical ventilator (2),
- or an NO/N₂/O₂ mixture via the secondary outlet (141) to a manual insufflator or BAVU.
